# EUROPEAN PATENT SPECIFICATION

(11) **EP 2 098 225 B1**
(45) Date of publication and mention of the grant of the patent: **09.03.2011**
(21) Application number: 09007900.5
(22) Date of filing: 04.01.2006
(51) Int. Cl.: A61K 9/58, A61K 9/50, A61K 9/16, A61K 9/26, A61K 9/20

(54) **Taste masking system for non-plasticizing drugs**
Geschmacksunterdrückungssystem für nicht weichgemachte Arzneimittel
Système de masquage de goût pour médicaments non plastifiés

(30) Priority: 06.01.2005 US 641807 P; 10.01.2005 US 642619 P
(43) Date of publication of application: 09.09.2009
(62) Divisional of application: 06717339.3
(73) Proprietor: CIMA LABS INC., Eden Prairie, MN 55344-9361 (US)
(72) Inventor: Habbib, Walid, Cincinnati Ohio, 45244 (US); Moe, Derek, Maple Grove MN 55311 (US)
(74) Representative: Maiwald Patentanwalts GmbH

(56) References cited:
- EP-A- 1 688 131
- WO-A-00/09090
- WO-A-00/30617
- WO-A-98/30209
- WO-A-02/096392
- WO-A-2004/060354
- US-A- 4 874 613
- US-A- 4 966 770
- US-A- 5 464 632
- KLANCKE J.: "Dissolution testing of orally disintegrating tablets" DISSOLUTION TECHNOLOGIES, May 2003 (2003-05), pages 6-8, XP002545423

## Description

### BACKGROUND OF THE INVENTION

There are many advantages to changing the delivery system and format of an established drug. Some drugs which are found only in swallow tablets may be difficult for patients to swallow, particularly the elderly and small children. Developing dosage forms that can readily disintegrate in the mouth of a patient is a tremendous advantage where possible. However, it is important that such dosage forms be organoleptically pleasant, i.e., do not provide a relatively gritty sensation so as to make their ingestion unpalatable. Moreover, tablets that disintegrate in the mouth often expose the patient to the taste of the active ingredient which, not infrequently, is dreadful.

Taste masking technologies are known. However, not all taste masking technologies can work with every drug. Various taste masking technologies can, in certain instances, interfere with disintegration, provide inadequate taste masking for a given active or, as importantly, interfere with the bioavailability or pharmacokinetic properties of the drug relative to a swallow tablet. In addition, designing and producing disintegrable dosage forms that are taste masked of ten can increase the expense of the dosage form when compared to merely directly compressing a tablet. Often these systems require coating operations and sometimes multiple coating operations, which can be difficult and expensive. It can also require that coating apparatus be cleaned between successive coating operations or that large capital expenditures be made to purchase two or more coating apparatus.

A system which would eliminate the need for multiple coating apparatus or cleaning of multiple apparatus would be a great advantage.

### SUMMARY OF THE INVENTION

One aspect of the invention is the mixing, dissolving or dispersing of a non-plasticizing active pharmaceutical ingredient ("API") directly with or in a taste masking material and using the resulting material as a taste masking coating. The resulting coating is also contemplated.

Another aspect of the present invention is a taste masked pharmaceutical formulation comprising a solid support, at least one API-containing layer which is covering at least a portion of the solid support, and at least one overcoating layer covering at least a portion of the API-containing layer. The solid support may be precoated with one or more layers over which are coated the API-containing layer(s). The API-containing layer comprises at least one API and at least one first taste masking material. The overcoating layer can be any material, but is preferably at least one of at least one second taste masking material or a material which is pH dependent and becomes soluble at a pH of about 6.5 or less. The taste masking coating and overcoating may be made from the same material, the latter not including the API.

In one aspect, the formulation or dosage form made therefrom also includes at least one additional ingredient generally mixed with or granulated with the taste masked particles. The additional ingredient is selected from the group consisting of binders, glidants, disintegrants, effervescent couples, colors, flavors, coatings, lubricants and carriers.

In one embodiment, the first and the second taste masking materials used in the API-containing layer and the overcoating layer respectively are composed of the same material. In one embodiment, both are composed of a polymer or copolymer whose solubility is pH dependent and which becomes soluble at a pH of about 6.5 or below, and more preferably about 6.0 or below, and most preferably, an acrylic polymer or copolymer.

In another aspect of the present invention, the overcoating layer does not include any active pharmaceutical ingredient, aside from any which may leach in, or will be disposed at the interface between the overcoating and the API-containing layers or which is present in incidental amounts, i.e., less than 3% of the total amount of API.

In another embodiment in accordance with the present invention, the taste masked formulation can include more than one API-containing layer and/or more than one overcoating layer. When a plurality of such layers are present, they may be layered in any order. For example, the solid support can be coated with an API-containing layer, which can in turn be coated with an overcoating layer, which can in turn be coated with an API-containing layer, which can in turn be coated with a second API-containing layer, and finally, a second overcoating layer. As another embodiment, the solid support can be coated with a first API-containing layer, a second API-containing layer and an overcoating layer coating at least a portion of said second API-containing layer. Where a plurality of API-containing layers are present, said layers may contain the same or different API(s) and/or taste masking material(s). As previously stated, the solid support may be coated with one or more undercoating layers prior to the application of the API-containing layer(s).

Another aspect of the present invention is a dosage form intended to be placed in the mouth and disintegrated/dissolved in the mouth before being swallowed. The dosage form in accordance with this aspect of the present invention includes a taste masked formulation as disclosed herein comprising a solid support, at least one API-containing layer covering at least a portion of the solid support and at least one overcoating layer covering at least a portion of the API-containing layer or may contain a plurality of layers as described previously. The dosage form also includes at least one additional ingredient in the form of a filler, lubricant, disintegrant, binder, glidant, effervescent couple, color, flavor, lubricant, coating and/or carrier. The resulting dosage forms are in the form of a tablet, capsule, caplet, gelcap, powder, gum, film, syrup, liquid or suspension.

In a preferred embodiment, the dosage form is a solid dosage form intended to disintegrate and/or dissolve in the mouth of a patient, preferably in a period of two minutes or less, more preferably 90 seconds or less, and even more preferably 60 seconds or less. This dosage form is often selected from tablets, capsules, caplets, gums and films.

In another aspect of the present invention there is provided a solid dosage form which is intended to disintegrate in the mouth of a patient, preferably in a period of two minutes or less, more preferably 90 seconds or less, and even more preferably 60 seconds or less. This dosage form is often selected from tablets, capsules, caplets, gums and films. In a particularly preferred embodiment, this rapid disintegration is achieved without loss of taste masking. Taste masking can be estimated by showing that a dosage form provides a release of not more than about 45% of its content of API within about 5 minutes when tested by a using a USP 2 paddle test as described herein in a media having a pH of about 6.8.

The present invention also provides various methods of making a taste masked formulation and/or dosage form. One such method comprises steps of mixing at least one non-plasticizing API with at least one first taste masking material and at least one solvent. This forms a first taste masking mixture. The solid support is then coated, at least in part, with the first taste masking mixture to form an API-containing layer and preferably allowed to dry. The solid support coated with the API-containing layer is then coated with an overcoating layer comprising a second taste masking mixture, which is itself comprised of at least one second taste masking material and at least one solvent to form an overcoating layer. Again, the first taste masking material and the second taste masking material may be the same or may be different. This process can be modified to include the application of more than one of each of the layers as described previously. The resulting taste masking materials may then be mixed with one or more additional ingredients and formed into a dosage form, such as, for example, being compressed into a tablet.

The present invention is directed to a taste masked formulation to treat or prevent a condition. Any active pharmaceutical ingredient that qualifies as non-plasticizing could be incorporated into formulations in accordance with the present invention. Such an API could be used in the formulations and dosage forms of the invention to treat, prevent or affect a condition in a patient for which that API is generally used or for which a doctor deems appropriate. The method entails administering to a subject in need of treatment or prevention of a condition, an orally disintegrable dosage form comprising at least one solid support, at least one API-containing layer covering at least a portion of the at least one solid support, and at least one overcoating layer covering at least a portion of the at least one API-containing layer, placing the orally disintegrable dosage form into the mouth of the subject, maintaining the dosage form in the mouth for a time which is sufficient to allow the dosage form, or portions thereof, to disintegrate and/or dissolve, and swallowing the resulting disintegrated and/or dissolved material. In a preferred embodiment, the at least one API-containing layer comprises alprazolam and at least one first taste masking material and the overcoating layer comprises at least one second taste masking material.

The dosage form preferably also includes at least one additional ingredient selected from the group consisting of binders, glidants, effervescent couples, colors, flavors, coatings, lubricants and carriers. In a particularly preferred embodiment the orally disintegrable dosage form is in the form of a compressed tablet which can disintegrate/dissolve in the mouth of a patient within about 2 minutes or has more preferably 90 seconds or less and most preferably 60 seconds or less.

In another embodiment, the process of treating patients also involves watching the patient for a period of time sufficient to ensure the disintegration of the dosage form and that the patient swallowed, thus reducing the possibility that the patient hid the dosage form in his/her mouth, only to spit it out when the health professional's back was turned. It is not necessary to watch the patient in all instances. Indeed, in accordance with another aspect there is provided a method of treating a patient in need thereof by placing a tablet in accordance with the present invention in the patient's mouth and allowing it to at least partially disintegrate and/or dissolve followed by swallowing with saliva. In a preferred embodiment, it is placed on the top of the tongue where it dissolves/disintegrates within a few seconds prior to being swallowed.

The dosage forms of the present invention may be swallowed with water. However, they are preferably orally disintegrable and water need not be taken.

The present invention provides numerous advantages. Eudragit E-100, for example, can be dissolved or dispersed in a number of solvents such as alcohol or water. This allows one to dissolve or suspend drugs which are, for example, water insoluble or incompatible. Spraying the resulting mixture onto the surface of a solid support helps reduce the overall exposed drug surface area, assisting in taste masking by reducing the degree of exposure. In addition, the Eudragit E-100 is capable of providing taste masking in and of itself. This further enhances the overall taste masking achieved in accordance with the present invention.

Not only does the Eudragit E-100 used in this type of formulation provide superior taste masking, it acts as a good binder and is relatively non-tacky and easily processed. This improves workability, content uniformity and the like. Moreover, because the taste masking coating used in the overcoating layer and the taste masking coating contained in the API layer can be made from the same material, one need not use a second coating apparatus or necessarily interrupt the process to clean and reconfigure for a coating using a separate material. Indeed, one can, without significant interruption, and even without drying, stop the feed of the API-containing material and begin feeding in the overcoating material. This can save considerable processing time without sacrificing performance.

In still another embodiment, there is provided an orally disintegrable tablet that can disintegrate in the mouth within about 90 seconds or less including, without limitation, the dosage forms described herein including a solid support, an API containing layer and an overcoating layer, and which provides a release of not more than about 45% of its content of API within about 5 minutes when tested by a USP 2 apparatus using a USP 2 paddle test as described herein in a media having a pH of about 6.8. In still another embodiment, there is provided an orally disintegrable tablet that can disintegrate in the mouth within about 90 seconds or less and which provides a release of not less than about 85% of its content of API within about 5 minutes when tested by a using a USP 2 paddle test as described herein in a media having a pH of about 6.0. In another embodiment, it will meet both of the above standards.

There is also provided a method of evaluating the taste masking ability of a formulation including but not limited to orally disintegrable and/or dissolvable formulations. In one embodiment, the method includes the step of testing a dosage form in a medium having a pH of about 6.8 using a USP 2 apparatus and a USP 2 paddle test and determining whether or not more than about 45% of the content of API is released within about 5 minutes.

In another embodiment, the API-containing layer material, e.g., the combination of the API and the first taste masking material, can be used as a granulation binder. The resulting granulate can be coated with the one or more overcoating layers directly or can first be coated with one or more API-containing layers prior to application of one or more overcoating layers. This formulation can then be mixed with one or more additional ingredients as described above and formulated into dosage forms.

In another embodiment, there is provided an API-containing orally disintegrable/dissolvable tablet ("ODT") tablet that provides adequate taste masking as measured by a bitterness analysis.

In another embodiment, there is provided a taste masked formulation comprising: a solid support, at least one non-plasticizing API-containing layer covering at least a portion of the solid support and at least one overcoating layer covering at least a portion of the non-plasticizing API-containing layer. The non-plasticizing API-containing layer comprises non-plasticizing API and at least one first taste masking material and the overcoating layer comprises at least one second taste masking material. In one aspect of this embodiment, the first and/or the second taste masking materials are pH dependent and become soluble at a pH of about 6.5 or less.

And in still another embodiment, there is provided a pharmaceutical dosage form comprising: a non-plasticizing API and at least a first taste masking material. The dosage form disintegrates in the mouth within about 90 seconds or less and provides a release of not more than about 45% of its content of the non-plasticizing API within about 5 minutes when tested by a USP 2 paddle test in a media having a pH of about 6.8 and provides a release of not less than about 85% of its content of the non-plasticizing API within about 5 minutes when tested by a USP 2 paddle test in a media having a pH of about 6.0.

In another embodiment, there is provided a pharmaceutical dosage form comprising: the non-plasticizing API and at least a first taste masking material. The orally disintegrable dosage form can disintegrate in the mouth within about 90 seconds or less. The at least one first taste masking material and is pH dependent and becomes soluble at a pH of about 6.5 or less.

### DETAILED DESCRIPTION

Throughout the entire specification, including the claims, the word "comprise" and variations of the word, such as "comprising" and "comprises," as well as "have," "having," "includes," "include" and "including," and variations thereof, means that the named steps, elements or materials to which it refers are essential, but other steps, elements or materials may be added and still form a construct with the scope of the claim or disclosure. When recited in describing the invention and in a claim, it means that the invention and what is claimed is considered to what follows and potentially more. These terms, particularly when applied to claims, are inclusive or open-ended and do not exclude additional, unrecited elements or methods steps. The term "between" as used in connection with a range includes the endpoints unless the context suggests otherwise. All references to testing is at room temperature (20-25°C) unless otherwise specified and all references to temperature are in degrees centigrade unless otherwise specified.

In the present context, "consisting essentially of" is meant to exclude any excipient or combination of excipients or, as appropriate, any amount of any excipient or combination of excipients, as well as any pH adjusting substance or any amount of pH adjusting substance that would alter the basic and novel characteristics of the invention.

A solid support in accordance with the present invention can be composed of any material useful for layering in accordance with this and other conventional pharmaceutical applications. These can include, without limitation, particles, crystals, granulates, capsules, microparticles, microgranules, microcrystals or microcapsules. Particles, granules and crystals have their traditional meaning. "Capsule" in accordance with the present invention includes generally hollow, spherical vessels such as liposomes, micelles and the like. These may be dried. Solid supports can be composed of any number of materials or mixtures thereof including particles created from one or more of the taste masking materials, polymers, solid dicalcium phosphate and the like. However, in a preferred embodiment, the solid supports are made of a sugar. "Sugar" in accordance with the present invention generally includes other forms of carbohydrate such as, for example, sugars, sugar alcohols, ketoses, saccharides, polysaccharides, oligosaccharides and the like, as well as celluloses and modified celluloses. These include, without limitation, sucrose, mannitol (spray dried and granular) lactose, and microcrystalline cellulose. Most preferred in accordance with the present invention are sucrose and microcrystalline cellulose. Useful sucrose spheres are available from Paulaur corporation, 105 Melrich Road, Cranbury, NJ 08512. Useful microcrystalline spheres are sold by Asahi Kasei Chemicals Corp, with the following address: Hibiya-Mitsui Building 1-2 Yurakucho 1-chome, Chiyoda-ku, Tokyo 100-8440 Japan under the designation CELPHERES.

The size of the solid support can vary considerably with, amongst other things, the application, volume of the solid support that will be used in the formulation, the type of dosage form in which it will be included, and the thicknesses of the layers that will coat it. Solid supports that are too small can be difficult to coat. Solid supports that are too large can be difficult to work with, can affect content uniformity and can provide an unpleasant organoleptic sensation in the mouth. Of course, the larger the particle size, the smaller the surface area of the API that will be provided in the mouth thus reducing the potential exposure to the taste buds and other sensory organs within the mouth, further enhancing taste masking. Size may also vary depending upon the use of undercoatings. Thus an undercoating layer of E-100 could be applied to the solid support prior to application of an API-containing layer.

In accordance with the present invention, the solid support size is preferably between about 10 microns and about 1,000 microns, more preferably between about 20 microns and 600 microns. This means that at least about 90% of the solid support, by weight, fall within these ranges based on sieving. In a more preferred embodiment, the solid support will predominantly have more than 50% fall within a 60 to 80 mesh screen cut. More particularly, the amount by weight greater than 300 µm is about 0%, the amount by weight greater than 250 µm is less than about 10%, the amount in between about 180 and about 250 µm is about 90% or more, and the amount by weight less than 180 µm is about 10% or less.

A 45-60 mesh screen cut with similar percentages may also be preferred. Again, about 90% of the particles should be between about 250 microns and about 350 microns. This is measured as before. "Micro" in the context of solid supports means a solid support having a particle size of below about 50 microns. Preferably the solid support is substantially spherical although the particle dimensions can vary and can be, without limitation, elliptical, generally egg-shaped, rod-shaped, regular and/or irregularly shaped.

Covering at least a portion of the solid support is at least one API-containing layer. By "covering at least a portion" in context of the API-containing layer, it is understood that the complete surface area of each particle as solid support need not be covered. Indeed, while the efficiency of the system is improved considerably by the use of substantially complete and uniform coating, thus reducing the number of solid support particles necessary to deliver a given amount of API, it is not required that the coating of the API-containing material cover even a majority of the particles of solid support or a majority of the surface of the solid support. Preferably, however, the API-containing layer covers substantially all of the solid support to which it is applied (it is possible to mix some coated and uncoated solid support if desired). By "substantially all" it is understood that, generally speaking, at least about 85% by weight of the coating material (the API and first taste masking material) used at the start of the coating process is actually coated onto the solid support. Thus, at least about 85% of the API coating layer material applied (API and first taste masking material) actually coats the solid support. Relatively little, therefore, is wasted.

The API-containing layer, and indeed the overcoating layer as well, can be applied by any normal process such as use of a Wurster fluidized bed where the coating material enters from the bottom of the reactor. When this process was used, it was found that 85% or more by weight of the API-containing coating could be applied to the solid support. For example, if 1 kilogram of coating were prepared and used in the process, at least 850 grams would actually end up on the solid support particles. The amount of API-containing coating material can also be calculated based on the weight gain of the solid support (including an undercoating - if any). Thus the amount of coating can result in a weight gain of between about 0.1 and about 300%, more preferably between about 1.0 and about 200% by weight of the API-containing coating relative to the weight of the solid support. This is based on the total amount of the API and the first taste masking material and does not include solvent or other coating additives.

The at least one first taste masking material useful in accordance with the present invention generally includes any natural or synthetic polymer including: acrylic polymers, modified celluloses, and the like, which are pH dependant materials that become soluble at a pH of about 6.5 or below, more preferably about 6.0 or below. These polymers and copolymers should preferably be pharmacologically acceptable, capable of providing appropriate release and effective taste masking while still being convenient to process. These include, for example, amino alkyl acrylate copolymers such as, for example, copolymers of methylmethacrylate, butylmethacrylate and dimethylaminoethyl methacrylate. See European Pharmacopoeia 4.4 (04/2003:1975) at 3385. In one particularly preferred embodiment, the copolymer has a relative molecular mass of about 150,000 and a ratio of dimethylaminoethyl methacrylate groups to butylmethacrylate groups and methylmethacrylate groups of about 2:1:1 and the content of the dimethylaminoethyl groups is about 20.8% to 25.5% based on the amount of dry substances present.

A particularly preferred material can be obtained under the mark Eudragit E-100, which can be used in normal form or in micronized Eudragit E-100 and mixtures thereof. Eudragit is a trademark of Rohm GmbH, Chemische Fabrik, Kirschenallee, D-64293, Darmstadt, Germany for a group of acrylic polymers.

These materials are generally solid at room temperature. However, they may be applied to the solid support and mixed with the API by being dissolved, suspended, emulsified, dispersed or the like in a solvent or solvent system. Preferred solvents in accordance with the present invention include those capable of substantially dissolving or dispersing Eudragit E-100 such as water, normal C₁-C₅ alcohol, branched C₁-C₅ alcohol, denatured C₁-C₅ alcohol, and low molecular weight ketones such as acetone and MEK. Ethanols, including (SDA-3A) and denatured ethanol are most preferred.

The active pharmaceutical ingredient useful in accordance with the present invention is one which has been found to be "non-plasticizing." This is a pharmaceutically active material that is relatively non-tacky and generally will remain relatively non-tacky so as to render coated solid supports workable when combined with the first taste masking material, whether or not up to about 25% by weight of a conventional anti-tack agent, such as talc or magnesium stearate is added. A "plasticizing" active pharmaceutical ingredient cannot meet this requirement. They will be relatively tacky and unworkable in an active pharmaceutical ingredient-containing layer, even with 25% of an anti-tacking agent. A particularly preferred API that is particularly well suited for use with this invention is alprazolam.

Indeed, it was found that when certain APIs were mixed with Eudragit E-100 and applied to sugar spheres, the results was a gummy, sticky mess that, when dried, could not be properly processed into uniform particles of the desired composition, nature and properties. The workability of this material was poor. Often plasticizing active pharmaceutical ingredients will not permit overcoating without any interruption as is the case with the preferred non-plasticizing active pharmaceutical ingredients of the invention.

Some APIs such as, for example, alprazolam, although sparingly soluble at working concentrations in ethanol, were found to coat quite nicely and allowed application of the overcoating layer in the same equipment without interruption other than that necessary to change the feed of coating material. The resulting particles were discreet, non-tacky and exceptionally workable. By working with various materials, and without wishing to be bound by a particular theory, it was determined that certain drugs react and/or interact with the polymer materials in the taste masking coating material, changing their individual characters rendering the material more tacky. The present invention intends to encompass only those active pharmaceutical ingredients ("APIs") that would not so adversely affect workability so as to prevent their effective use in forming discrete, preferably free flowing, well coated, well characterized solid supports, which may be further coated. Active pharmaceutical ingredients that may be used in accordance with the present invention may include, without limitation, analgesic, anti-inflammatories, antipyretics, antibiotics, antimicrobials, anxiolytics, laxatives, anorexics, antihistamines, antidepressants, antiasthmatics, antidiuretics, antiflatuents, antimigraine agents, antispasmodics, sedatives, antihyperactives, antihypertensives, tranquilizers, decongestants, beta blockers, peptides, proteins, oligonucleotides and other substances of biological origin, and combinations thereof. Also contemplated are the drugs and pharmaceutically active ingredients described in Mantelle, U.S. Pat. No. 5,234,957, in columns 18 through 21. That text of Mantelle is hereby incorporated by reference. The above-identified APIs, however, are limited to those which are substantially non-plasticizing as defined herein.

The amount of solvent used in forming the API-containing coating will depend on, among other things, the taste masking coating material used. Moreover, more solvent may be needed to achieve dissolution than dispersion, for example. However, since the solvent is generally removed by drying, it should not make up an appreciable portion of the final product (generally less than 5% total, preferably less than 3% and more preferably less than 1% total) and therefore, the amount of solvent is not generally considered in describing the overall composition of the API-containing layer or for that matter, the overcoating layer. The amount of the API in the API-containing layer can vary from between about 0.1% to about 90% by weight of said API-containing layer. More preferably the amount ranges from between about 1% to about 75% by weight. The API-containing layer may also include anti-tack agents such as magnesium stearate or talk and copolymers such as HPMC, EC, HPC and PVP in an amount of up to about 25% by weight of that coating.

The amount of API used in each dosage form in accordance with the present invention will vary. However, generally, the taste masked dosage forms in accordance with the present invention will provide a dose of API between about 0.10 micrograms and about 2 grams, preferably between about 0.50 micrograms and about 1 gram per dosage form (e.g., tablet, teaspoonful, etc.), most preferably between about 10 micrograms to about 0.5 grams.

The overcoating layer can be any material that meets the criteria of the invention. However, in a preferred embodiment, it is either a materials which becomes soluble at a pH of about 6.5 or below or comprises at least a second taste masking material. It is possible and indeed often is the case that this material is both. Indeed, this taste masking material, like the first taste masking material, can generally be any polymeric material that can effectively taste mask the API and becomes soluble at a pH of about 6.5 or below, more preferably 6.0 or below, as previously described. More preferably, the second taste masking material is selected from the same group of materials previously identified for the first taste masking material including Eudragit E-100. In a particularly preferred embodiment, the second taste masking material is identical to the first taste masking material. Thus, both the API-containing layer and the overcoating layer may be made from the same polymeric material.

The overcoating layer covers at least a portion of the API-containing layer. "Covering at least a portion of" in the context of the overcoating layer means that an effective portion of the surface area of the solid support coated with the API-containing layer is itself covered so as to effectively provide taste masking. The adequacy and completeness of the coating can be measured by weight increase as previously suggested herein so long as the resulting material provide adequate taste masking. Without limitation, one way to test in vitro whether or not a formulation will likely have adequate taste masking is to measure by dissolution. A dissolution of 45% or less at 5 min. at pH 6.8 as described herein can serve as a viable model in some instances. Indeed, the fact that the release is less than 45% under these conditions alone suggest the overall adequacy of both the API-containing coating and the overcoating. Preferably, "substantially all" of the API-containing layer is coated with the overcoating which, in the context of the overcoating layer, means that at least about 85% of the coating material used, the second taste masking material (without considering any solvent or additive), actually coats the API-containing-layer-coated solid support. The amount of overcoating material can also be calculated based on the weight gain of the solid support which has been coated with the first taste-masking coating. Thus the amount of coating can result in a weight gain of between about 0.1 and about 300%, more preferably between about 1.0 and about 200% by weight of the overcoating relative to the weight of the solid support and first taste-masking coating. This is based on the total amount of the overcoating material and does not include solvent or other coating additives. In a particularly preferred embodiment, the amount of each coating layer ranges from about 1 to about 50% based on the weight of the solid support or solid support and first taste-masking layer as appropriate.

In the alternative, the total amount of all coating materials used can range from about 0.2 to about 1200% by weight based on the initial weight of the solid support, more preferably, from about 1 to about 700%, even more preferably from about 1 to about 500%, still more preferably, from about 1 to about 600% and most preferably from about 2 to about 400%.

In another embodiment, the combination of the alprazolam-containing layer and the overcoating layer are able to provide taste masking as measured by drug release under specified conditions. Specifically, solid dosage forms made from the taste masked formulations of the invention can be tested using a USP 2 paddle method (50 r.p.m.) in 500 mL of phosphate buffered water at pH of 6.8 and 37°C. This is referred to herein as the "USP 2 paddle test." Generally, if amount of drug released under these conditions after five minutes is 45% or less, suitable taste masking has been achieved. See Tables 1 and 2 below. Preferably release is less than 35% in five minutes. With particularly bad tasting drugs, the drug release after five minutes should be no more than about 30% weight of the alprazolam. Indeed, in some embodiments, it may be necessary or desirable that the percent release in 5 minutes at pH 6.8 is no more than about 25% by weight and in still another embodiment, not more than about 20% by weight.

**TABLE 1: % Release of Alprazolam 1/10th Scale Registration* Batch Tablets in pH 6.8 Dissolution Medium**

| | Sample ID | | | |
|---|---|---|---|---|
| | 0.25 mg¹ | 0.5 mg² | 1 mg³ | 2 mg⁴ |
| Time Point (min) | Average % Released (n=3) | | | |
| 2 | NT | 8 | 12 | NT |
| 5 | 20 | 19 | 19 | 15 |
| 10 | 41 | 40 | 41 | 35 |
| 15 | 55 | 54 | 52 | 45 |
| 30 | 74 | 73 | 67 | 62 |

| | | | | |
|---|---|---|---|---|
| NT=not tested *Registration Batch Tablets tested were stored for 27M @ ambient storage conditions | | | | |

**TABLE 2: % Release of Alprazolam Full Scale Batch Tablets in pH 6.8 Dissolution Medium**

| | Sample ID | | | | |
|---|---|---|---|---|---|
| | 0.25mg¹ | 0.5mg² | 1mg³ | 2mg⁴ | 2mg⁴ |
| Time Point (min) | Average % Released (n=3) | | | | |
| 2 | 5 | 11 | 6 | 3 | 5 |
| 5 | 14 | 19 | 14 | 7 | 12 |
| 10 | 35 | 35 | 28 | 16 | 26 |
| 15 | 52 | 49 | 41 | 24 | 39 |
| 30 | 75 | 70 | 63 | 40 | 59 |

| | | | | | |
|---|---|---|---|---|---|
| ¹ 0.25 mg tablets had a formulation such as that described generally in example 2. ² 0.50 mg tablets had a formulation such as that described generally in example 3. ³ 1.0 mg tablets had a formulation such as that described generally in example 4. ⁴ 2.0 mg tablets had a formulation such as that described generally in example 5. | | | | | |

All dissolution samples were prepared using plastic syringes, pretreated PE filter tips, and 13-mm diameter, 0.45 µm, GHP syringe filters. Approximately 2mL of the sample aliquot was filtered through the GHP syringe filter prior to collection in the HPLC vial.

**Table 3. %Release of 0.25 mg Alprazolam Tablets**

| (10 kg batch size using different mesh size coated AL) (pH 6.8 Medium)¹ | | | | |
|---|---|---|---|---|
| | 0.25 mg (Control) | 0.25 mg (Coated AL passed through a 40 mesh) | 0.25 mg (Coated AL passed through a 45 mesh) | 0.25 mg (Coated AL passed through a 50 mesh) |
| Time Point (min) | Average % Released (n=3) | | | |
| 2 | 7 | 8 | 8 | 11 |
| 5 | 16 | 20 | .19 | 23 |
| 10 | 38 | 41 | 43 | 47 |
| 15 | 54 | 58 | 60 | 63 |

**Table 4 % Release of Alprazolam 1/10^{th} Scale Registration Tablets**

| (pH 6.8 Medium) | | | | |
|---|---|---|---|---|
| | 0.25 mg¹ | 0.5 mg² | 1. mg³ | 2 mg⁴ |
| Time Point (min) | Average % Released (n=3) | | | |
| 2 | NT | 8 | 12 | NT |
| 5 | 20 | 19 | 19 | 15 |
| 10 | 41 | 40 | 41 | 35 |
| 15 | 55 | 54 | 52 | 45 |
| 30 | 74 | 73 | 67 | 62 |

| | | | | |
|---|---|---|---|---|
| NT = Not Tested | | | | |

**Table 5. % Release of Alprazolam Full Scale Alprazelam™ Tablets**

| (Validation and Commercial) | | | | | |
|---|---|---|---|---|---|
| (pH 6.8 Medium) | | | | | |
| | 0.25 mg¹ | 0.5 mg² | 1 mg³ | 2 mg⁴ | 2 mg⁴ |
| Time Point (min) | Average % Released (n=3) | | | | |
| 2 | 5 | 11 | 6 | 3 | 5 |
| 5 14 | | | 19 14 | 7 | 12 |
| 10 | 35 | 35 | 28 | 16 | 26 |
| 15 | 52 | 49 | 41 | 24 | 39 |
| 30 | 75 | 70 | 63 | 40 | 59 |

| | | | | | |
|---|---|---|---|---|---|
| Dissolution Results in pH 6.8 Medium | | | | | |

**Table 6. %Release of Commercial Xanax Tablets**

| (pH 6.8 Medium) | | |
|---|---|---|
| | 0.25 mg Xanax, Lot# 23DYS | 2 mg Xanax, Lot# 92HKB |
| Time Point (min) | Average % Released (n=3) | |
| 2 | 56 | 25 |
| 5 | 79 | 72 |
| 10 | 91 | 93 |
| 15 | 94 | 96 |

In one preferred embodiment, both the API-containing layer and the overcoating layer include at least one polymeric material that is common to both. In a most preferred embodiment, the at least one second taste masking material used in the overcoating layer is identical to the at least one first taste masking material used in the API-containing layer. In one embodiment, both are Eudragit E-100. In another most preferred embodiment the API is alprazolam.

Either layer may be made of a mixture of taste masking materials where none, some or all of the material used in each layer are the same or different. The overcoating layer of the taste masked formulations of the invention can be produced by dissolving or dispersing the second taste masking material in at least one solvent, as was previously described in the context of the first taste masking mixture to form a second taste masking mixture. The at least one solvent is preferably the same as those previously described in connection with the API-containing layer. This material is then coated on top of the at least one first API-containing layer to form an overcoating layer. Again, preferably, after applied, the at least one solvent would be removed, preferably by drying. In one preferred embodiment, however, there is no need to dry the API-containing layer before application of the overcoating layer. Indeed, most preferably, there is no need to interrupt the coating process, or even clean or change apparatus. One need only discontinue application of the first taste masking material and API and may, immediately if desired, begin application of the second taste masking material.

In an alternate embodiment, the material making up the API containing layer (the at least one API and the at least one first taste masking material) can act as a binder for wet granulation. Thus, as mentioned previous, at least one API is mixed with at least one first taste masking material producing a first taste masking mixture. This first taste masking mixture is then in one embodiment, mixed or blended with at least one additional ingredient which acts as a support. Preferably the at least one additional ingredient is chosen from among the solid supports previously described or "sugar" as previously defined. By blending the combination of the first taste masking mixture and the at least one additional ingredient, granules are produced.

Said granules may be coated with an overcoating layer comprising a second taste masking mixture comprising at least one second taste masking material. Alternatively, or additionally as a separate overcoating layer, the overcoating layer can comprise a third taste masking mixture comprising at least one API and at least one third taste masking material. It is understood that multiple overcoating layers, in any combination of second or third taste masking material and using any combination of APIs, may be employed in coating the granule. Thus, for purposes of example only, the granule could first be coated with the second taste masking mixture and the coated with the third taste masking mixture. Alternatively, again for purposes of example only, the granule could first be coated with the third taste masking mixture, coated a second time with the third taste masking mixture, coated a third time with the second taste masking mixture, and coated a fourth time with the third taste masking mixture. It is preferable for the at least one second and/or third taste masking material to be the same as the at least one first taste masking material. A solvent may also be included in any of the first, second, and/or third taste masking mixtures. Where such a solvent is used it is preferred that the solvent used in the second and/or third taste masking mixture to be the same as that used in the first taste masking mixture. It is preferred that where said granules are coated with the second and/or third taste masking mixture that they be substantially coated and, ideally, that they be totally coated. After coating the granules, if a solvent is used in any of the first, second and/or third taste masking mixtures, it is preferred to remove the solvent by drying. However, if no solvent is used then drying is unnecessary. The granules formed in this manner may then be incorporated in dosage forms as described herein.

Dosage forms in accordance with the present invention are preferably solid dosage forms which are designed to disintegrate and/or dissolve rapidly in the mouth of a patient once placed in his mouth. By "rapidly," it is understood that these dosage forms preferably disintegrate in the mouth in less than 120 seconds, more preferably 90 second or less, even more preferably 60 seconds or less, and most preferably 45 seconds. "Disintegration" in this context refers to the break up of the tablet into constituent particles. Note that the taste masked formulation (the taste masked beads or granulate) in accordance with the present invention should not dissolve or disintegrate, as individual units, to any discernable degree (as established in a bitterness test or otherwise) during the time that they are within the mouth. That is to say while the dosage form may disintegrate and/or portions of it may dissolve in the mouth, the taste masked particles should largely remain intact while in the mouth. It is also possible that some or all of the additional ingredients contained within the dosage form will disintegrate and/or dissolve within the period of time prescribed. Dissolution in accordance with the present invention means that the material will actually be soluble in saliva as opposed to merely breaking down to constituent particles.

Solid dosage forms in accordance with the present invention include tablets, capsules, caplets, gels and films, as well as powders. While orally disintegrable solid dosage forms are preferred in accordance with the present invention, the present invention also encompasses the use of the taste masked formulations in accordance with the present invention in liquids, syrups and suspensions. Of course, to do so, at least the overcoating layer must be insoluble in the liquid carrier used for the formulation.

The dosage forms may include as additional ingredients or excipients glidants, fillers, lubricants, binders, sweeteners, disintegrants, flavoring and coloring components. Any conventional sweetener or flavoring component may be used. Combinations of sweeteners, flavoring components, or sweeteners and flavoring components may likewise be used.

An effervescent couple, alone or in combination with other ingredients may be used to improve the disintegration profile and the organoleptic properties of the dosage form. Effervescent couples are made from a reaction of a soluble acid source and a metal carbonate or bicarbonate. The acid sources or acid may be any which are safe for human consumption and may generally include food acids, acid anhydrides and acid salts. Food acids include citric acid, tartaric acid, malic acid, fumaric acid, adipic acid, and succinic acids etc. Because these acids are directly ingested, their overall solubility in water is less important than it would be if the effervescent tablet formulations of the present invention were intended to be dissolved in a glass of water. Acid anhydrides and acid salts of the above described acids may also be used. Acid salts may include sodium, dihydrogen phosphate, disodium dihydrogen pyrophosphate, acid citrate salts and sodium acid sulfite.

Carbonate sources include dry solid carbonate and bicarbonate salts such as sodium bicarbonate, sodium carbonate, potassium bicarbonate and potassium carbonate, magnesium carbonate and sodium sesquicarbonate, sodium glycine carbonate, L-lysine carbonate, arginine carbonate and amorphous calcium carbonate. These effervescent couples may be provided in an amount of between about 3% and about 25% by weight of the dosage form.

In addition to the effervescence-producing agents, a dosage form according to the present invention may also include, instead of or in addition thereto, suitable non-effervescent disintegration agents. Non-limiting examples of non-effervescent disintegration agents include: microcrystalline, cellulose, croscaramellose sodium, crospovidone, starches, corn starch, potato starch and modified starches thereof, clays, such as bentonite, alginates, gums such as agar, guar, locust bean, karaya, pecitin and tragacanth. These non-effervescent disintegrants may comprise up to about 20 weight percent and preferably between about 2% and about 10% of the total weight of the dosage form.

Examples of binders which can be used include but are not limited to acacia, tragacanth, gelatin, starch, cellulose materials such as methyl cellulose, microcrystalline cellulose and sodium carboxy methyl cellulose, alginic acids and salts thereof, magnesium aluminum silicate, polyethylene glycol, PVP, guar gum, polysaccharide acids, bentonites, sugars, invert sugars and the like. Binders may be used in an amount of up to 60 weight percent and preferably about 10 to about 40 weight percent of the total dosage form.

Coloring agents may include but are not limited to titanium dioxide, and dyes suitable for food such as those known as F.D.& C. dyes and natural coloring agents such as grape skin extract, beet red powder, beta-carotene, annato, carmine, turmeric, paprika, etc. The amount of coloring used may range from about 0.1 to about 3.5 weight percent of the total dosage form.

Examples of glidants include but are not limited to silicon dioxide, talc, calcium stearate, magnesium stearate, stearowet C, zinc stearate, calcium silicate, starch, pregelatinized starch, magnesium lauryl sulfate, magnesium carbonate, magnesium oxide, and others These may be used in an amount of between about 0.1 and about 5% by weight of the dosage form.

Diluents or Fillers include, but are not limited to spray-dried monohydrate or anhydrous lactose, sucrose, dextrose, mannitol, sugar alcohols, sorbitol, starch, cellulose (e.g., microcrystalline cellulose) dihydrated or anhydrous dibasic calcium phosphate, tricalcium phosphate, maltodextrins, calcium carbonate, calcium sulfate and others. These may be used in an amount of between about 10 and about 90% by weight of the dosage form.

Examples of carriers include liquid sugar, syrup, water and the like. Examples of disintegrants include but are not limited to starches, clays, microcrystalline celluloses, celluloses, algins, gums or cross linked polymers, .PVP-XL, sodium starch glycolate and croscarmellose sodium, and effervescent agents. Effervescent agents include but are not limited to: the acid sources or acid may be any which are safe for human consumption and may generally include food acids, acid anhydrides and acid salts. Food acids include citric acid, tartaric acid, malic acid, fumaric acid, adipic acid, and succinic acids etc. Acid anhydrides and acid of the above described acids may also be used. Acid salts may include sodium, dihydrogen phosphate, disodium dihydrogen pyrophosphate, acid citrate salts and sodium acid sulfite. Carbonate sources include dry solid carbonate and bicarbonate salts such as sodium bicarbonate, sodium carbonate, potassium bicarbonate and potassium carbonate, magnesium carbonate and sodium sesquicarbonate, sodium glycine carbonate, L-lysine carbonate, arginine carbonate and amorphous calcium carbonate.

Flavors incorporated in the composition may be chosen from synthetic flavor oils and flavoring aromatics and/or natural oils, extracts from plants, leaves, flowers, fruits and so forth and combinations thereof. These may include cinnamon oil, oil of wintergreen, peppermint oils, clove oil, bay oil, anise oil, eucalyptus, thyme oil, cedar leave oil, oil of nutmeg, oil of sage, oil of bitter almonds and cassia oil. Also useful as flavors are vanilla, citrus oil, including lemon, orange, grape, lime and grapefruit, and fruit essences, including apple, pear, peach, strawberry, raspberry, cherry, plum, pineapple, apricot and so forth. Flavors which have been found to be particularly useful include commercially available orange, grape, cherry and bubble gum flavors and mixtures thereof. The amount of flavoring may depend on a number of factors, including the organoleptic effect desired. Flavors may be present in an amount ranging from about 0.05% to about 3% by weight based upon the weight of the dosage form.

Lubricants may also be used. Hydrophobic lubricants are preferred. Hydrophobic lubricants include, without limitation, calcium stearate, magnesium stearate, zinc stearate, stearic acid, stearowet C, mineral oil, vegetable oil, glyceryl behenate, sodium stearyl fumarate, talc, starch, and others. Hydrophilic lubricants include, without limitation, sodium benzoate, sodium chloride, sodium lauryl sulfate, magnesium lauryl sulfate, polyethylene glycol, and others. Magnesium stearate is preferred. These may be used in an amount of between about 0.5% and about 5% by weight, more preferably 0.5% to about 2.5% by weight of the dosage form. If desired the dosage form may also contain minor amounts of nontoxic substances such as wetting or emulsifying agents, pH buffering agents and the like, for example, sodium acetate, sorbitan monolaurate, triethanolamine, sodium acetate, triethanolamine oleate, sodium lauryl sulfate, dioctyl sodium sulfosuccinate, polyoxyethylene sorbitan fatty acid esters.

The dosage forms in accordance with the present invention preferably have a hardness of at least about 5 Newtons and are designed to disintegrate rapidly in the mouth of a patient in less than about 2 minutes, preferably 90 seconds, to thereby liberate the taste masked formulations of the invention. Preferably the dosage form will disintegrate in less than 60 seconds and even more preferably 45 seconds. This measure of hardness is based on the use of small tablets of less than about 0.25 inches in diameter. A hardness of at least about 10 Newtons is preferred for larger tablets. Most preferably, however, the dosage forms in accordance with the present invention have a hardness of between about 10 and about 150 Newtons and, more preferably, between about 10 and about 120 Newtons. Proportionate hardnesses are expected for tablets of various sizes.

When the dosage forms in accordance with the present invention are tablets, they are preferably sufficiently robust that they can be tabletted using conventional tabletting and handling equipment, as well as packaged in traditional multi-tablet bottles. See U.S. Pat. No. 6,024,981. These tablets preferably have a hardness of at least about 15 Newtons and most preferably a friability of less than 2% when measured by U.S.P., more preferably less than 1% when measured by U.S.P. Most preferably the tablets in accordance with this aspect of the invention have a hardness of between about 15 and about 100 Newtons and a friability of 1% or less when measured by U.S.P. See again U.S. Pat. No. 6,024,981.

Tablets can either be manufactured by direct compression, wet granulation, dry granulation or any other tablet manufacturing technique. See, e.g., U.S. Pat. Nos. 5,178,878 and 5,223,264, which are incorporated by reference herein. Other dosage forms in accordance with the present invention can be made in their traditional manner using the taste masking formulation as a part of their components. Liquid forms can be made by dispersing, suspending, emulsifying, or forming a colloid of the particles of the taste mask formulation of the present invention in one or more conventional delivery vehicles.

The formulations and dosage forms of the present invention are useful for treating or preventing any condition for which administration of the API contained therein is considered an appropriate treatment or preventative measure. Thus the present invention includes a method of treating a condition in a subject wherein said condition is treatable with an API. This method includes the following steps: administering to the subject an orally disintegrable tablet comprising, at least one solid support, at least one API-containing layer covering the at least one solid support, and at least one overcoating layer covering at least a portion of the at least one API-containing layer, placing said orally disintegrable tablet into the mouth of the subject, maintaining the tablet in the mouth of the subject for a time which is sufficient to allow the tablet to disintegrate and/or dissolve, and swallowing the resulting disintegrated and/or dissolved tablet. The formulation used should include an amount of API which is effective to treat or prevent the condition for which it is prescribed or administered. It is preferred that the at least one API-containing layer comprise an API and at least one first taste masking material and that the at least one overcoating layer comprising at least one second taste masking material. The dosage form may also include at least one additional ingredient selected from the group consisting of binders, glidants, effervescent couples, color, flavors, coatings, lubricants, and carriers. It is also preferred that the orally disintegrable dosage form be in the form of a compressed tablet which can disintegrate in the mouth of a patient within about 60 seconds. In a preferred embodiment, the tablet is placed on top of the tongue and allowed to disintegrate/dissolve and the than swallowed. The patient may be watch for a time sufficient to ensure that the tablet has been dissolved and swallowed.

The dosage forms of the present invention may be swallowed with water. However, they are preferably orally disintegrable and water need not be taken.

In still another embodiment, there is provided an orally disintegrable dosage form including at least one API and in a preferred embodiment, at least one taste masking material (first taste masking material) that can disintegrate in the mouth within about 90 seconds or less and which provides a release of not more than 45% of its content of API within 5 minutes when tested by a USP 2 apparatus using a USP 2 paddle test as described herein in a media having a pH of 6.8. This can be accomplished with the multi-layered dosage forms described herein or the granulated dosage forms. However, other techniques are also contemplated so long as they provide this same result.

In still another embodiment, there is provided an orally disintegrable tablet that can disintegrate in the mouth within about 90 seconds or less and which provides a release of not less than 85% of its content of API within 5 minutes when tested by a using a USP 2 paddle test as described herein in a media having a pH of 6.0. In still a further embodiment, there is provided an orally disintegrable tablet that can disintegrate in the mouth within about 90 seconds or less and which provides a release of not less than 90% of its content of API within 5 minutes when tested by a using a USP 2 paddle test as described herein in a media having a pH of 6.0. The pH testing at pH 6 is based on a an average of several tests. Again, this can be accomplished with the multi-layered dosage forms described herein or the granulated dosage forms. However, other techniques are also contemplated so long as they provide this same result.

In a particularly preferred embodiment, there is provided an orally disintegrable tablet that can disintegrate in the mouth within about 90 seconds or less and which provides a release of not more than 35% of its content of API within 5 minutes in a media having a pH of 6.8 when tested by a using a USP 2 paddle test as described. Again, this can be accomplished with the multi-layered dosage forms described herein or the granulated dosage forms. However, other techniques are also contemplated so long as they provide this same result.

In another embodiment, the API-containing layer material, e.g., the combination of the API and the first taste masking material, can be used as a granulation binder. Granulation can be wet or dry granulation and can be accomplished using any known granulation technique. While it is possible to granulate the API directly, usually a support or filler, such as microcrystalline cellulose or mannitol, or a combination of fillers and excipients as described herein, may be used in the granulation process with the polymer/API solution acting as binder/granulation liquid. Sufficient amounts of each ingredient should be used to assure proper particle size distribution and content uniformity. The resulting granulate can be coated with the one or more overcoating layers directly or can first be coated with one or more API-containing layers prior to application of one or more overcoating layers as described previously for the solid support in the non-granulated aspects of the invention. The resulting granulate and/or coated granulate can next be tabletted directly, mixed with other additional ingredients as described herein or otherwise formed into a dosage form as described herein. Being metered into a capsule or directly compressed into a tablet as a dried granulate are preferred.

The relative proportion of the non-plasticizing API and first taste masking material in the granulation is the same as that previously described for the layered sold support embodiments described herein.

EXAMPLES

Example 1

| Coated Alprazolam 2.57% | | | |
|---|---|---|---|
| **COMPONENT NAME** | **PRODUCTION FORMULA (kg)** | **MATERIAL FORMULA (mg/g)** | **FOOT NOTES** |
| Alprazolam, USP | 5.141 | 25.67 | |
| Sugar Spheres, NF | 143.00 | 714.09 | 1 |
| Eudragit E-100, EP/JPE | 40.4 | 201.7 | |
| Magnesium Stearate, NF/EP/JP | 11.714 | 58.50 | 2 |
| Alcohol, SDA-3A, Anhydrous | 272.6 | N/A | 3 |
| **TOTAL** | 200.255 | 1000.00 | |

| | | | |
|---|---|---|---|
| Footnotes: 1 60/80 Grade 2 Non-Bovine grade 3 Alcohol is removed during processing | | | |

Example 2

| 0.25 mg Alprazolam, 1/4", Orange Flavor, Yellow Tablets | |
|---|---|
| **COMPONENT NAME** | **QUANTITY (mg/tablet)** |
| Alprazolam, Coated ¹ | 9.73 |
| Mannitol | 76.07 |
| Disintegrants/binder | 11.00 |
| Magnesium Stearate, NF/BP/JP | 1.50 |
| Natural & Artificial Flavor | 0.75 |
| Sucralose, NF | 0.50 |
| Colloidal Silicon Dioxide, NF/EP | 0.30 |
| Ferric Oxide, NF | 0.15 |
| **TOTAL** | 100.0 |

| | |
|---|---|
| Footnotes: 1 Amount based on theoretical potency of 2.57% | |

Example 3

| 0.5 mg Alprazolam, 5/16", Orange Flavor, Yellow Tablets | |
|---|---|
| **COMPONENT NAME** | **QUANTITY (mg/tablet)** |
| Alprazolam, Coated ¹ | 19.46 |
| Mannitol | 152.14 |
| Disintegrants/binder | 22.00 |
| Magnesium Stearate, NF/EP/JP | 3.00 |
| Natural & Artificial Flavor | 1.50 |
| Sucralose, NF | 1.00 |
| Colloidal Silicon Dioxide, NF/EP | 0.60 |
| Ferric oxide, NF | 0.30 |
| **TOTAL** | 200.0 |

| | |
|---|---|
| Footnotes: 1 Amount based on theoretical potency of 2.57% | |

Example 4

| 1.0 mg Alprazolam, 5/16", Convex, Orange Flavor, White Tablets | |
|---|---|
| **COMPONENT NAME** | **QUANTITY (mg/tablet)** |
| Alprazolam, Coated ¹ | 38.91 |
| Mannitol | 132.99 |
| Mannitol, USP/EP/JP | 50.00 |
| Disintegrants/binder | 22.00 |
| Magnesium Stearate, NF/EP/JP | 3.00 |
| Natural & Artificial Flavor | 1.50 |
| Sucralose, NF | 1.00 |
| Colloidal Silicon Dioxide, NF/EP | 0.60 |
| **TOTAL** | 200.0 |

| | |
|---|---|
| Footnotes: 1 Amount based on theoretical potency of 2.57% | |

Example 5

| 2.0 mg Alprazolam, 3/8", Convex, Orange Flavor, White Tablets | |
|---|---|
| **COMPONENT NAME** | **QUANTITY (mg/tablet)** |
| Alprazolam, Coasted ¹ | 77.82 |
| Mannitol | 265.98 |
| Disintegrants/binder | 44.00 |
| Magnesium Stearate, NP/EP/JP | 6.00 |
| Natural & Artificial Flavor | 3.00 |
| Sucralose, NF | 2.00 |
| Colloidal Silicon Dioxide, NF/EP | 1.20 |
| **TOTAL** | 400.0 |

| | |
|---|---|
| Footnotes: 1 Amount based on theoretical potency of 2.57% | |

Example 6
Dissolution of tablets produced generally in accordance with Examples 1-5 can be tested using a standard USP dissolution apparatus 2 with a paddle speed of 50 rpm, in 250 mL of 70 mM phosphate buffer pH 7.4. The analysis is performed by HPLC.

### Results

| | | % Released (minutes) | | | | |
|---|---|---|---|---|---|---|
| Tablet strength (mg) | # of tablets/vessel | 1 minute | 2 minutes | 3 minutes | 4 minutes | 5 minutes |
| 0.25 | 8 | 2.54 | 3.96 | 5.23 | 6.23 | 7.23 |
| 0.5 | 4 | 2.49 | 3.87 | 4.94 | 5.81 | 6.57 |
| 1 | 2 | 2.35 | 3.78 | 4.80 | 5.64 | 6.50 |
| 2 | 1 | 2.32 | 3.82 | 5.21 | 6.19 | 6.87 |

| | | | | | | |
|---|---|---|---|---|---|---|
| Note: These tests reflect batches made of tablets generally falling within the formulations reflected in Examples 2-5. Only one vessel was tested for each strength. This was not the aforementioned test for taste masking. It shows the release profile of tablets in accordance with the present invention other. | | | | | | |

Example 7

| 0.25 mg Alprazolam Tablet Analysis | | | | | |
|---|---|---|---|---|---|
| Release Testing | | | | | |
| Test Method | Claim Specifications | Results | | | |
| Physical Appearance | Yellow, convex, round beveled edge scored tablets. Confirm debossing "SP 321" on one side and "0.25" on the other. | Yellow, convex, round beveled edge scored tablets. Confirm debossing "SP 321" on one side and "0.25" on the other. | | | |
| Identity by HPLC | Positive for alprazolam | Conforms | | | |
| Assay | 90.0%-110.0% Label Claim | 101.1% | | | |
| Dissolution | Report Released at 5, 15, 30, and 45 min. | % Released (n=12) | | | |
| | NLT 80% (Q) at 30 minutes | 5 min | 15 min | 30 | min 45 min |
| | | 96 | 98 | 99 | 99 |
| | | | | Range: 91-112 | |
| Disintegration | Report mean value and the Range: | Avg. = 23 seconds | | | |
| | | Low =20 High = 30 | | | |
| Water Content | Report Value | 0.48% | | | |
| Hardness | Report Value | 28 N | | | |

Dissolution was tested using the following apparatus and procedure. This testing procedure was also used for examples 8-20. The dissolution of tablets reported in examples 7-20 used tablets produced generally in accordance with examples 1-5. These were not tests of the aforementioned test for taste masking.

### Parameters:

| | | |
|---|---|---|
| 1. | Instrumentation: | Dissolution system |
| | Apparatus: | USP 2, paddles |
| | Medium: | 70 mM potassium phosphate buffer, pH 6.0 |
| | Medium Volume: | 500 mL |
| | Medium Temperature: | 37.0 *C * 0.5 *C |
| | Paddle Speed: | 50 rpm |
| 2. | Instrumentation: | HPLC system with UV detector |
| | Separation: | Reversed-phase, pH 3.0 buffer and acetonitrile |
| | Detection: | 254 nm |

Example 8

| 0.25 mg Alprazolam Tablet Analysis | | | | | |
|---|---|---|---|---|---|
| Release Testing | | | | | |
| Test Method | Claim Specifications | Results | | | |
| Physical Appearance | Yellow, convex, round beveled edge scored tablets. Confirm debossing "SP 321" on one side and "0.25" on the other. | Yellow, convex, round beveled edge scored tablets. Confirm debossing "SP 321" on one side and "0.25" on the other. | | | |
| Identity by HPLC | Positive for alprazolam | Conforms | | | |
| Assay | 90.0%-110.0% Label Claim | 97.8% | | | |
| Dissolution | Report % Released at 5, 15, 30, and 45 min. NLT 80% (Q) at 30 minutes | % Released (n=12) | | | |
| | | 5 min | 15 min | 30 min | 45 min |
| | | 95 | 98 | 98 Range: 89-106 | 98 |
| Disintegration | Report mean value and the Range: | Avg. = 21 seconds | | | |
| | | Low = 16 High = 25 | | | |
| Water Content | Report Value | 0.47% | | | |
| Hardness | Report Value | 27 N | | | |

Example 9

| 0.5 mg Alprazolam Tablet Analysis | | | | | |
|---|---|---|---|---|---|
| Release Testing | | | | | |
| Test Method | Claim Specifications | Results | | | |
| Physical Appearance | Yellow, convex, round beveled edge scored tablets. Confirm debossing "SP 322" on one side and "0.5" on the other. | Yellow, convex, round beveled edge scored tablets. Confirm debossing "SP 322" on one side and "0.5" on the other. | | | |
| Identity by HPLC | Positive for alprazolam | Conforms | | | |
| Assay | 90.0%-110.0% Label Claim | 101.6% | | | |
| Dissolution | Report % Released at 5, 15, | % Released (n=12) | | | |
| | 30, and 45 min. NLT 80% (Q) at 30 minutes | 5 min | 15 min | 30 min | 45 min |
| | | 96 | 99 | 101 | 101 |
| | | | | Range: 90-111 | |
| Disintegration | Report mean value and the Range: | Avg. = 28 seconds | | | |
| | | Low = 21 High = 36 | | | |
| Water Content | Report Value | 0.47% | | | |
| Hardness | Report Value | 29 N | | | |

Example 10

| 0.5 mg Alprazolam Tablet Analysis | | | | | |
|---|---|---|---|---|---|
| Release Testing | | | | | |
| Test Method | Claim Specifications | Results | | | |
| Physical Appearance | Yellow, convex, round beveled edge scored tablets. Confirm debossing "SP 322" on one side and "0.5" on the other. | Yellow, convex, round beveled edge scored tablets. Confirm debossing "SP 322" on one side and "0.5" on the other. | | | |
| Identity by HPLC | Positive for alprazolam | Conforms | | | |
| Assay | 90.0%-110.0% Label Claim | 99.6% | | | |
| Dissolution | Report % Released at 5, 15, 30, and 45 min. NLT 80% (Q) at 30 minutes | % Released (n=12) | | | |
| | | 5 min | 15 min | 30 min | 45 min |
| | | 95 | 100 | 100 | 100 |
| | | | | Range: 93-108 | |
| Disintegration | Report mean value and the Range: | Avg. = 25 seconds | | | |
| | | Low = 18 High = 35 | | | |
| Water Content | Report Value | 0.45% | | | |
| Hardness | Report Value | 30 N | | | |

Example 11

| 0.25 mg Alprazolam Tablet Analysis | | | | | |
|---|---|---|---|---|---|
| Test Method | Claim Specifications | Results | | | |
| Physical Appearance | Yellow, convex, round beveled edge scored tablets. Confirm debossing "SP 321" on one side and "0.25" on the other. | Yellow, convex, round beveled edge scored tablets. Confirm debossing "SP 321" on one side and "0.25" on the other. | | | |
| Identity by HPLC | Positive for alprazolam | Conforms | | | |
| Assay | 90.0%-110.0% Label Claim | 101.1% | | | |
| Dissolution | Report % Released at 5, 15, | % Released (n=12) | | | |
| | 30, and 45 min. NLT 80% (Q) at 30 minutes | 5 min | 15 min | 30 min | 45 min |
| | | 96 | 98 | 99 | 99 |
| | | | | Range: 91-112 | |
| Disintegration | Report mean value and the Range: | Avg. = 23 seconds | | | |
| | | Low = 20 High = 30 | | | |
| Water Content | Report Value | 0.56% | | | |
| Hardness | Report Value | 28 N | | | |

Example 12

| 0.25 mg Alprazolam Tablet Analysis | | | | | |
|---|---|---|---|---|---|
| Release Testing | | | | | |
| Test Method | Claim Specifications | Results | | | |
| Physical Appearance | Yellow, convex, round beveled edge scored tablets. Confirm debossing "SP 321" on one side and "0.25" on the other. | Yellow, convex, round beveled edge scored tablets. Confirm debossing "SP 321" on one side and "0.25" on the other. | | | |
| Identity by HPLC | Positive for alprazolam | conforms | | | |
| Assay | 90.0%-110.0% Label Claim | 97.8% | | | |
| Dissolution | Report % Released at 5, 15, 30, and 45 min. NLT 80% (Q) at 30 minutes | % Released (n=12) | | | |
| | | 5 min | 15 min | 30 min | 45 min |
| | | 95 | 98 | 98 Range : 89-106 | 98 |
| Disintegration | Report mean value and the Range: | Avg. = 21 seconds | | | |
| | | Low =16 High = 25 | | | |
| Water Content | Report Value | 0.56% | | | |
| Hardness | Report Value | 27 N | | | |

Example 13

| 0.5 mg Alprazolam Tablet Analysis | | | | | |
|---|---|---|---|---|---|
| Release Testing | | | | | |
| Test Method | Claim Specifications | Results | | | |
| Physical Appearance | Yellow, convex, round beveled edge scored tablets. Confirm debossing "SP 322" on one side and "0.5" on the other. | Yellow, convex, round beveled edge scored tablets. Confirm debossing "SP 322" on one side and "0.5" on the other. | | | |
| Identity by HPLC | Positive for alprazolam | Conforms | | | |
| Assay | 90.0%-110.0% Label Claim | 101.6% | | | |
| Dissolution | Report % Released at 5, 15, 30, and 45 min. NLT 80% (Q) at 30 minutes at | % Released (n=12) | | | |
| | | 5 min | 15 min | 30 min | 45 min |
| | | 96 | 99 | 101 | 101 |
| | | | | Range: 90-111 | |
| Disintegration | Report mean value and the Range: | Avg. = 28 seconds | | | |
| | | Low = 21 High = 36 | | | |
| Water Content | Report Value | 0.63% | | | |
| Hardness | Report Value | 29 N | | | |

Example 14

| 0.5 mg Alprazolam Tablet Analysis | | | | | |
|---|---|---|---|---|---|
| Release Testing | | | | | |
| Test Method | Claim specifications | Results | | | |
| Physical Appearance | Yellow, convex, round beveled edge scored tablets. Confirm debossing "SP 322" on one side and "0.5" on the other. | Yellow, convex, round beveled edge scored tablets. Confirm debossing "SP 322" on one side and "0.5" on the other. | | | |
| Identity by HPLC | Positive for alprazolam | Conforms | | | |
| Assay | 90.0%-110.0% Label Claim | 99.6% | | | |
| Dissolution | Report % Released at 5, 15, 30, and 45 min. NLT 80% (Q) at 30 minutes | % Released (n=12) | | | |
| | | 5 min | 15 min | 30 min | 45 min |
| | | 95 | 100 | 100 | 100 |
| | | | | Range: 93-108 | |
| Disintegration | Report mean value and the Range: | Avg. = 25 seconds | | | |
| | | Low = 18 High = 35 | | | |
| Water Content | Report Value | 0.61% | | | |
| Hardness | Report Value | 30 N | | | |

Example 15

| 1 mg Alprazolam Tablet Analysis | | | | | |
|---|---|---|---|---|---|
| Release Testing | | | | | |
| Test Method | Claim Specifications | Results | | | |
| Physical Appearance | White, convex, round beveled edge scored tablets. Confirm debossing "SP 323" on one side and "1" on the other. | White, convex, round beveled edge scored tablets. Confirm debossing "SP 323" on one side and "1" on the other. | | | |
| Identity by HPLC | Positive for alprazolam | Conforms | | | |
| Assay | 90.0%-110.0% Label Claim | 98.7% | | | |
| Dissolution | Report % Released at 5, 15, 30, and 45 min. NLT 80% (Q) at 30 minutes | % Released (n=12) | | | |
| | | 5 min | 15 min | 30 min | 45 min |
| | | 92 | 98 | 99 Range: 95-105 | 99 |
| Disintegration | Report mean value and the Range: | Avg. = 25 seconds | | | |
| | | Low = 20 High = 31 | | | |
| Water Content | Report Value | 0.62% | | | |
| Hardness | Report Value | 30 N | | | |

Example 16

| 1 mg Alprazolam Tablet Analysis | | | | | |
|---|---|---|---|---|---|
| Release Testing | | | | | |
| Test Method | Claim Specifications | Results | | | |
| Physical Appearance | white, convex, round beveled edge scored tablets. Confirm debossing "SP 323" on one side and "1" on the other. | White, convex, round beveled edge scored tablets. Confirm debossing "SP 323" on one side and "1" on the other. | | | |
| Identity by HPLC | Positive for alprazolam | Conforms | | | |
| Assay | 90.0%-110.0% Label Claim | 100.7% | | | |
| Dissolution | Report % Released at 5, 15, 30, and 45 min. NLT 80% (Q) at 30 minutes | % Released (n=12) | | | |
| | | 5 min 15 | min | 30 min | 45 min |
| | | 94 | 98 | 99 Range: 94-107 | 99 |
| Disintegration | Report mean value and the Range: | Avg. = 26 seconds | | | |
| | | Low = 21 High = 35 | | | |
| Water Content | Report Value | 0.64% | | | |
| Hardness | Report Value | 31 N | | | |

Example 17

| 2 mg Alprazolam Tablet Analysis | | | | | |
|---|---|---|---|---|---|
| Release Testing | | | | | |
| Test Method | claim Specifications | Results | | | |
| Physical Appearance | White, convex, round beveled edge scored tablets. Confirm debossing "SP 324" on one side and "2" on the other. | White, convex, round beveled edge scored tablets. Confirm debossing "SP 324" on one side and "2" on the other. | | | |
| Identity by HPLC | Positive for alprazolam | Conforms | | | |
| Assay | 90.0%-110.0% Label Claim | 99.7% | | | |
| Dissolution | Report % Released at 5, 15, 30, and 45 min. NLT 80% (Q) at 30 minutes | % Released (n=12) | | | |
| | | 5 min | 15 min | 30 min | 45 min |
| | | 94 | 99 | 99 | 100 |
| | | | | Range: 96-107 | |
| Disintegration | Report mean value and the Range: | Avg. = 35 seconds | | | |
| | | Low = 26 High = 40 | | | |
| Water Content | Report Value | 0.60% | | | |
| Hardness | Report Value | 35 N | | | |

Example 18

| 2 mg Alprazolam Tablet Analysis | | | | | |
|---|---|---|---|---|---|
| Release Testing | | | | | |
| Test Method | Claim Specifications | Results | | | |
| Physical Appearance | White, convex, round beveled edge scored tablets. Confirm debossing "SP 324" on one side and "2" on the other. | White, convex, round beveled edge scored tablets. Confirm debossing "SP 324" on one side and "2" on the other. | | | |
| Identity by HPLC | Positive for alprazolam | Conforms | | | |
| Assay | 90.0%-110.0% Label Claim | 101.9% | | | |
| Dissolution | Report % Released at S, 15, 30, and 45 min. NLT 80% (Q) at 30 minutes | % Released (n=12) | | | |
| | | 5 min | 15 min | 30 min | 45 min |
| | | 92 | 101 | 101 | 102 |
| | | | | Range: 97-109 | |
| Disintegration | Report mean value and the Range: | Avg. = 35 seconds | | | |
| | | Low = 31 High = 41 | | | |
| Water Content | Report Value | 0.58% | | | |
| Hardness | Report Value | 36 N | | | |

Example 19

| 0.5 mg Alprazolam Flat Faced Tablet Analysis | | | | | |
|---|---|---|---|---|---|
| Testing | | | | | |
| Test Method | Claim Specifications | Results | | | |
| Physical Appearance | Yellow, flat faced, round beveled edge scored tablets. Confirm debossing "SP 322" on one side and "0.5" on the other. | Complies | | | |
| Identity by HPLC | Positive for alprazolam | Complies | | | |
| Assay | 90.0%-110.0% Label Claim | 101.3% label claim | | | |
| Dissolution | (Q) = 80% at 30 minutes 1) NMT 0 less than 85% (n=6) 2) Average NLT 80%; NMT 0 < 65% (N=12) 3) Average NLT 80%; NMT 2 < 65%, and NMT 0 < 55% (n=24) | Avg. (30 min) = 102% Released | | | |
| | | Vessel | (%) | Vessel | (%) |
| | | 1 | 99 | 7 | 108 |
| | | 2 | 99 | 8 | 103 |
| | | 3 | 106 | 9 | 92 |
| | | 4 | 108 | 10 | 102 |
| | | 5 | 104 | 11 | 104 |
| | | 6 | 102 | 12 | 99 |
| | | Avg. (10 min) = 101% Released⁵ | | | |
| | | Vessel | (%) | Vessel | (%) |
| | | 1 | 97 | 7 | 106 |
| | | 2 | 96 | 8 | 101 |
| | | 3 | 105 | 9 | 91 |
| | | 4 | 107 | 10 | 100 |
| | | 5 | 103 | 11 | 102 |
| | | 6 | 101 | 12 | 97 |
| Water Content | NMT 2.5% | 0.65% | | | |
| Disintegration Time | Average NMT 60 seconds | Average (n=18) = 28 seconds | | | |

| | | | | | |
|---|---|---|---|---|---|
| ⁵ The 10 minute dissolution results are for information only. | | | | | |

Example 20

| 0.5 mg Alprazolam Flat Faced Tablet Analysis | | | | | |
|---|---|---|---|---|---|
| Testing | | | | | |
| Test Method | Claim Specifications | Results | | | |
| Physical Appearance | Yellow, flat faced, round beveled edge scored tablets. Confirm debossing "SP 321" on one side and "0.25" on the other. | Complies | | | |
| Identity by HPLC | Positive for alprazolam | Complies | | | |
| Assay | 90.0%-10.0% Label Claim | 101.6% label claim | | | |
| Dissolution | (Q) = 80% at 30 minutes 1) NMT 0 less than 95% (n=6) 2) Average NLT 80%; NMT 0 < 65% (N=12) 3) Average NLT 80%; NMT 2 < 65%, and NMT 0 < 55% (n=24) | Avg. (30 min) = 102% Released | | | |
| | | vessel | (%) | Vessel | (%) |
| | | 1 | 93 | 7 | 96 |
| | | 2 | 101 | 8 | 104 |
| | | 3 | 113 | 9 | 97 |
| | | 4 | 104 | 10 | 103 |
| | | 5 | 109 | 11 | 102 |
| | | 6 | 100 | 12 | 101 |
| | | Avg. (10 min) = 101% Released⁶ | | | |
| | | Vessel | (%) | Vessel | (%) |
| | | 1 | 92 | 7 | 96 |
| | | 2 | 100 | 8 | 104 |
| | | 3 | 112 | 9 | 95 |
| | | 4 | 102 | 10 | 102 |
| | | 5 | 109 | 11 | 100 |
| | | 6 | 100 | 12 | 101 |
| Water Content | NMT 2.5% | 0.68% | | | |
| Disintegration Time | Average NMT 60 seconds | Average (n=18) = 36 seconds | | | |

| | | | | | |
|---|---|---|---|---|---|
| ⁶ The 10 minute dissolution results are for information only. | | | | | |

Example 21
2.5 mg/g Batch Manufacturing
This process is used with the formulation described in examples 1 and 2.
The same process may be used generally for all of the formulations of examples 1-5.
Ethanol is used to dissolve the Eudragit E-100 which is mixed until dissolution is complete. Magnesium stearate is mixed in until homogeneous followed by addition of alprazolam. Mixing continues until homogenous. Sucrose spheres are charged to a Wurtzer fluid bed reactor and coated with the homogenous mixture including alprazolam to form the alprazolam-containing layer. For the overcoating layer, ethanol is used to dissolve the Eudragit E-100 which is mixed until dissolution is complete. Magnesium stearate is mixed in until homogeneous. This is coated over the alprazolam-containing coating on the sugar spheres using the same reactor, without emptying the reactor. The particles are screened and mixed with the other materials, then the lubricant is added.

## Claims

1. An orally disintegrable tablet that disintegrates in the mouth within about 90 seconds or less, said dosage form comprising a solid support, at least one API-containing layer covering at least a portion of said solid support, an undercoating layer coated onto said solid support, over which said API-containing layer is coated, at least one overcoating layer covering at least a portion of said API-containing layer, and at least one additional ingredient, said API-containing layer comprising at least one API and at least one first taste masking material, wherein the taste masking material includes an acrylic polymer or a modified cellulose, and wherein said dosage form provides a release of not more than 45% of its content of API within 5 minutes when tested by a USP 2 apparatus using a USP 2 paddle test as described herein in a media having a pH of 6.8.

2. The tablet according to claim 1, which provides a release of not less than 90% of its content of API within 5 minutes when tested by using a USP 2 paddle test as described herein in a media having a pH of 6.0.

3. The tablet according to claim 1 or claim 2 wherein said API is a non-plasticizing API.

4. The tablet according to claim 1, wherein said solid support is a particle, crystal, granule, capsule, microparticle, microgranule, microcrystal or microcapsule.

5. The tablet according to claim 4, wherein said solid support comprises sugar.

6. The tablet according to any one of claims 1 to 3 further comprising at least one additional ingredient selected from the group consisting of binders, glidants, disintegrants, effervescent couples, colors, flavors and carriers granulated with said at least one API and said at least one taste masking material.

7. The tablet according to claim 6, further comprising at least one overcoating layer wherein said overcoating layer covers at least a portion of said granulate.

8. The tablet according to any one of the preceding claims where in said taste masking material is an acrylic polymer.

9. The tablet according to claim 8, wherein said acrylic polymer is an amino alkyl acrylate copolymer.

10. The tablet according to any one of the preceding claims, wherein said API is selected from the group consisting of analgesics, anti-inflammatories, antipyretics, antibiotics, antimicrobials, anxiolytics, laxatives, anorexics, antihistamines, antidepressants, antiasthmatics, antidiuretics, antiflatuents, antimigraine agents, antispasmodics, sedatives, antihyperactives, antihypertensives, tranquilizers, decongestants, beta blockers, peptides, proteins, oligonucleotides and other substances of biological origin, and combinations thereof.

11. The tablet according to claim 9 wherein said API is alprazolam.

12. The tablet according to claim 1 wherein the overcoating layer comprises a second taste masking material that is the same as said first taste masking material.

## Patentansprüche

1. Im Mund zerfallende Tablette, die im Mund innerhalb von etwa 90 Sekunden oder weniger zerfällt, wobei die Dosierungsform einen festen Träger, wenigstens eine API-haltige Lage, die wenigstens einen Teil des festen Trägers bedeckt, eine auf den festen Träger aufgetragene Unterschichtlage, auf der die API-haltige Lage aufgetragen ist, wenigstens eine Überschichtlage, die wenigstens einen Teil der API-haltigen Lage bedeckt und wenigstens einen zusätzlichen Inhaltsstoff umfasst, wobei die API-haltige Lage wenigstens einen API und wenigstens ein erstes geschmacksmaskierendes Material umfasst, wobei das geschmacksmaskierende Material ein Acrylpolymer oder eine modifizierte Cellulose einschließt, und wobei die Dosierungsform bei einem Test mit einem USP-2-Apparat unter Anwendung eines wie hier beschriebenen USP-2-Paddle-Tests in einem Medium mit einem pH-Wert von 6,8 innerhalb von 5 Minuten nicht mehr als 45% seines Gehalts an API freisetzt.

2. Tablette nach Anspruch 1, die bei einem Test unter Anwendung eines wie hier beschriebenen USP-2-Paddle-Tests in einem Medium mit einem pH-Wert von 6,0 innerhalb von 5 Minuten nicht mehr als 90% ihres Gehalts an API freisetzt.

3. Tablette nach Anspruch 1 oder 2, wobei es sich bei dem API um einen nicht weichmachenden API handelt.

4. Tablette nach Anspruch 1, wobei es sich bei dem festen Träger um ein Partikel, einen Kristall, ein Granulat, eine Kapsel, ein Mikropartikel, ein Mikrogranulat, einen Mikrokristall oder eine Mikrokapsel handelt.

5. Tablette nach Anspruch 4, wobei der feste Träger Zucker umfasst.

6. Tablette nach einem der Ansprüche 1 bis 3, wobei die Tablette weiterhin wenigstens einen zusätzlichen Inhaltsstoff ausgewählt aus der Gruppe bestehend aus Bindemitteln, Gleitmitteln, Sprengmitteln, Brausepaaren, Farbstoffen, Geschmacksstoffen und Trägem, granuliert mit dem wenigstens einen API und dem wenigstens einen geschmacksmarkierenden Material, umfasst.

7. Tablette nach Anspruch 6, die weiterhin wenigstens eine Überschichtlage umfasst, wobei die Überschichtlage wenigstens einen Teil des Granulats bedeckt.

8. Tablette nach einem der vorhergehenden Ansprüche, wobei es sich bei dem geschmacksmaskierenden Material um ein Acrylpolymer handelt.

9. Tablette nach Anspruch 8, wobei es sich bei dem Acrylpolymer um ein Aminoalkylacrylat-Copolymer handelt.

10. Tablette nach einem der vorhergehenden Ansprüche, wobei der API ausgewählt ist aus der Gruppe bestehend aus Analgetika, entzündungshemmenden Mitteln, Antipyretika, Antibiotika, antimikrobiellen Mitteln, Anxiolytika, Abführmitteln, Anorexika, Antihistaminika, Antidepressiva, Antiasthmatika, Antidiuretika, Mitteln, die Blähungen verhindern, Antimigränemitteln, Antispasmodika, Beruhigungsmitteln, Mitteln gegen Hyperaktivität, blutdrucksenkenden Mitteln, Tranquilizern, Abschwellungsmitteln, Beta-Blockern, Peptiden, Proteinen, Oligonukleotiden und anderen Substanzen biologischen Ursprungs und Kombinationen davon.

11. Tablette nach Anspruch 9, wobei es sich bei dem API um Alprazolam handelt.

12. Tablette nach Anspruch 1, wobei die Überschichtlage ein zweites geschmacksmaskierendes Material umfasst, bei dem es sich um das gleiche Material wie bei dem ersten geschmacksmaskierenden Material handelt.

## Revendications

1. Comprimé oral délitable qui se délite dans la bouche en environ 90 secondes ou moins, ladite forme galénique comprenant un support solide, au moins une couche incluant le principe actif pharmaceutique couvrant au moins une partie dudit support solide, une sous-couche enrobant ledit support solide, par-dessus laquelle ladite couche incluant le principe actif pharmaceutique est déposée, au moins une surcouche couvrant au moins une partie de ladite couche incluant le principe actif pharmaceutique, et au moins un composant supplémentaire, ladite couche incluant le principe actif pharmaceutique comprenant au moins un principe actif pharmaceutique et au moins une première substance masquant le goût, la substance masquant le goût incluant un polymère acrylique ou une cellulose modifiée, et ladite forme galénique permettant d'obtenir une libération de moins de 45 % de sa teneur en principe actif pharmaceutique dans les 5 minutes dans un essai sur un dispositif USP 2 avec le protocole USP 2 à pales comme décrit dans la présente invention dans un milieu de pH 6,8.

2. Comprimé conforme à la revendication 1, qui permet d'obtenir une libération d'au moins 90 % de sa teneur en principe actif pharmaceutique dans les 5 minutes dans un essai avec le protocole USP 2 à pales comme décrit dans la présente invention dans un milieu de pH 6,0.

3. Comprimé conforme à la revendication 1 ou la revendication 2 où ledit principe actif pharmaceutique est un principe actif pharmaceutique non plastifiant.

4. Comprimé conforme à la revendication 1, où ledit support solide est une particule, un cristal, un granule, une capsule, une microparticule, un microgranule, un microcristal ou une microcapsule.

5. Comprimé conforme à la revendication 4, où ledit support solide comprend un sucre.

6. Comprimé conforme à l'une quelconque des revendications 1 à 3, comprenant en outre au moins un composant supplémentaire choisi dans le groupe constitué par les agents liants, les agents glissants, les agents délitants, les couples effervescents, les colorants, les arômes et les vecteurs, granulé avec au moins un principe actif pharmaceutique et le ou lesdites substances masquant le goût.

7. Comprimé conforme à la revendication 6, comprenant en outre au moins une surcouche, ladite surcouche couvrant au moins une partie dudit granulat.

8. Comprimé conforme à l'une quelconque des revendications précédentes, où ladite substance masquant le goût est un polymère acrylique.

9. Comprimé conforme à la revendication 8, où ledit polymère acrylique est un copolymère amino-alkyl-acrylate.

10. Comprimé conforme à l'une quelconque des revendications précédentes où ledit principe actif pharmaceutique est choisi dans le groupe constitué par les substances suivantes : analgésiques, anti-inflammatoires, antipyrétiques, antibiotiques, antimicrobiens, anxiolytiques, laxatifs, anorexiques, antihistaminiques, antidépresseurs, antiasthmatiques, antidiurétiques, antiflatulents, agents antimigraineux, antispasmodiques, sédatifs, antihyperactifs, antihypertenseurs, tranquillisants, décongestionnants, bêtabloquants, peptides, protéines, oligonucléotides et autres substances d'origine biologique, et leurs combinaisons.

11. Comprimé conforme à la revendication 9, où ledit principe actif pharmaceutique est l'alprazolam.

12. Comprimé conforme à la revendication 1 où la surcouche comprend une seconde substance masquant le goût qui est la même que la première substance masquant le goût.
